# EUROPEAN PATENT APPLICATION

(11) **EP 2 371 403 A2**
(43) Date of publication of application: **05.10.2011**
(21) Application number: 11158657.4
(22) Date of filing: 17.03.2011
(51) Int. Cl.: A61L 31/10, A61L 31/14

(54) **Guidewire**

(30) Priority: 31.03.2010 JP 2010080081
(71) Applicant: Asahi Intecc Co., Ltd., Nagoya-shi, Aichi 463-0024 (JP)
(72) Inventor: Satozaki, Junji, Nagoya-shi Aichi 463-0024 (JP)
(74) Representative: Winter, Brandl, Fürniss, Hübner, Röss, Kaiser, Polte - Partnerschaft

(57) **Abstract**

An object of the present invention is to provide a guidewire in which the movement of coils of strand is not inhibited by a hydrophilic coating agent, and flexibility of a coiled body is secured even in a case where its coiled body is coated with the hydrophilic coating agent. The guidewire includes a core shaft, a coiled body including coils of strand wound around an outer circumference of the core shaft, and a hydrophilic coating agent coating at least a part of the coiled body. At least the part of the coiled body is coated with the hydrophilic coating agent so that a gap may be formed between the coils of strand.

## Description

### Cross Reference to Related Applications

This application is based on Japanese Patent Application No. 2010-080081 filed with the Japan Patent Office on March 31, 2010, the entire content of which is hereby incorporated by reference.

### Technical Field

The present invention relates to a guidewire forming a lubricating surface.

### Background Art

Conventionally, as for a guidewire that guides a catheter or the like used by being inserted in a tubular organ such as a vessel, a digestive tract, or a ureter or an intracorporeal tissue for treatment or examination, several kinds each provided on its surface with a hydrophilic coating agent to reduce a friction with the intracorporeal tissue are proposed.

For example, in a medical instrument described in Japanese Patent Application Laid-Open No. 2004-215710, a lubricating coat 7 (a hydrophilic coating agent) is provided at the outer circumference of the entire instrument including a metal-made core wire 4 and coils (a first coil 5 and a second coil 6) provided at the end portion of the core wire 4.

Also, in a medical insertion instrument described in Japanese Patent Application Laid-Open No. 2008-237621, a hydrophilic polymer layer 3B (a hydrophilic coating agent) is provided on the surface of metal coils 2 wound around a tip portion of a wire main body 4 to be closely attached to each other.

### Summary of Invention

However, in the medical instrument merely provided with the hydrophilic coating agent as described in Japanese Patent Application Laid-Open No. 2004-215710 or 2008-237621, the hydrophilic coating agent may flow between coils of strand forming the coil, which may inhibit the movement of the coils of strand. Also, a part provided with the hydrophilic coating agent may be hardened. In this case, flexibility of the tip portion of the guidewire is impaired, and thus the guidewire may perforate a vessel or the like at the time of operating the guidewire.

The present invention has been made to solve the foregoing technical problems, and an object of the present invention is to provide a guidewire in which the movement of coils of strand is not inhibited, and flexibility of a coiled body is secured even in a case of being coated with a hydrophilic coating agent.

This object is solved by a guidewire having the features of claim 1.

A guidewire according to the invention includes a core shaft, a coiled body including coils of strand wound around an outer circumference of the core shaft, and a hydrophilic coating agent coating at least a part of the coiled body, wherein at least the part of the coiled body is coated with the hydrophilic coating agent so that adjacent coils of strand coated with the hydrophilic coating agent are spaced apart from each other by a gap which prevents a contact of the coated surfaces of the adjacent coils of strand.

It is to be noted that each coil of strand of the coils of strand forming the coiled body means a 360° turn or winding of the strand.

Further, according to the invention the coils of strand forming the coiled body may be wound around the outer circumference of the core shaft with a defined pitch assuring that adjacent coils of strand are spaced apart by a gap even after having been coated with the hydrophilic coating agent.

Advantageous embodiments are subject matter of dependent claims.

### Brief Description of Drawings

The foregoing and other objects, features, aspects and advantages of the invention will become more apparent from the following detailed description when taken in conjunction with the accompanying drawings.
Fig. 1 is a vertical cross-sectional view showing a guidewire according to a first embodiment of the present invention.
Fig. 2 is a partially enlarged view of coils of strand coated with a hydrophilic coating agent of the guidewire according to the first embodiment of the present invention.
Fig. 3 is a partially enlarged view of the coils of strand coated with a hydrophilic coating agent of a guidewire according to a second embodiment of the present invention.
Fig. 4 shows a modification example of the second embodiment.

### Description of Embodiments

Preferred embodiments of the present invention will be described below with reference to the accompanying drawings, in which like reference characters designate similar or identical parts throughout the several views thereof.
<1> A guidewire according to a first aspect includes a core shaft, a coiled body including coils of strand wound around an outer circumference of the core shaft, and a hydrophilic coating agent coating at least a part of the coiled body, wherein at least the part of the coiled body is coated with the hydrophilic coating agent so that adjacent coils of strand coated with the hydrophilic coating agent are spaced apart from each other by a gap which prevents a contact of the coated surfaces of the adjacent coils of strand.
<2> A guidewire according to a second aspect is one in which, in the guidewire according to the first aspect, a center of a combined cross-section formed by the coil of strand and the hydrophilic coating agent is displaced further in a radial outward direction of the coiled body than a center of a cross-section of the coil of strand coated with the hydrophilic coating agent, as such.
<3> A guidewire according to a third aspect is one in which, in the guidewire according to the first or second aspect, a thickness of the hydrophilic coating agent coating the coil of strand in an axial direction of the coiled body (a side surface direction of the coil of strand) is smaller than a thickness of the hydrophilic coating agent in a radial outward direction of the coiled body (an outward surface direction of the coil of strand).
<4> A guidewire according to a fourth aspect is one in which, in the guidewire according to any one of the first to third aspects, a thickness of the hydrophilic coating agent coating the coil of strand in a radial inward direction of the coiled body (an inward surface direction of the coil of strand) is smaller than the thickness of the hydrophilic coating agent in an axial direction of the coiled body (a side surface direction of the coil of strand).
<1> As described above, in the guidewire according to the first aspect, at least the part of the coiled body is coated with the hydrophilic coating agent so that the gap is formed between adjacent coils of strand. Accordingly, in this guidewire, the movement of the coils of strand is not inhibited, and flexibility of the coiled body is secured even in a case where the coils of strand are coated with the hydrophilic coating agent.
<2> In the guidewire according to the second aspect, the center of the combined cross-section formed by the coil of strand and the hydrophilic coating agent is displaced further in the radial outward direction of the coiled body than the center of the cross-section of the coil of strand coated with the hydrophilic coating agent, as such. Accordingly, durability of lubricity of the coiled body by the hydrophilic coating agent can be improved while flexibility of the coiled body is secured.
<3> In the guidewire according to the third aspect, the thickness of the hydrophilic coating agent coating the coil of strand in the axial direction of the coiled body (side surface direction of the coil of strand) is smaller than the thickness of the hydrophilic coating agent in the radial outward direction of the coiled body (outward surface direction of the coil of strand). Accordingly, the gap between the adjacent coils of strand can be still larger. This enables the adjacent coils of strand to be moved more freely. Also, in a case where the adjacent coils of strand come into contact with each other by bending the coiled body, portions each provided with the thin hydrophilic coating agent abut on each other. Accordingly, flexibility of the coiled body is secured further reliably.
<4> In the guidewire according to the fourth aspect, the thickness of the hydrophilic coating agent coating the coil of strand in the radial inward direction of the coiled body (inward surface direction of the coil of strand) is smaller than the thickness of the hydrophilic coating agent in the axial direction of the coiled body (side surface direction of the coil of strand). Thus, a space between the core shaft and the coiled body can be secured sufficiently. Accordingly, flexibility of the coiled body can be further improved.

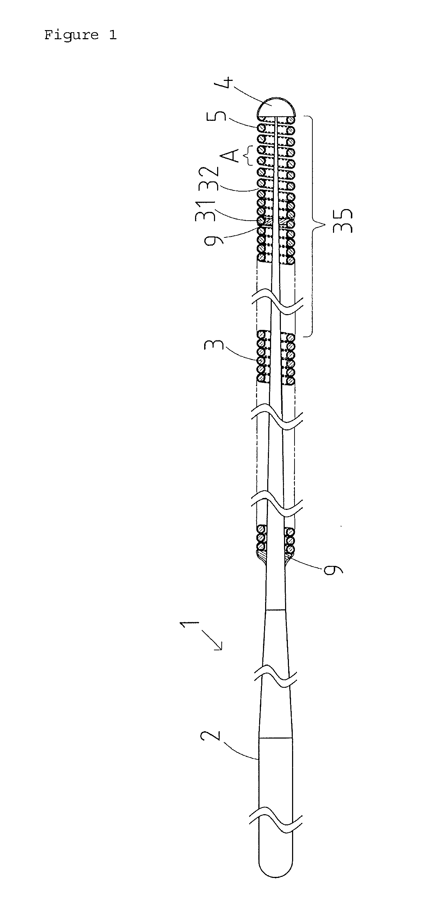

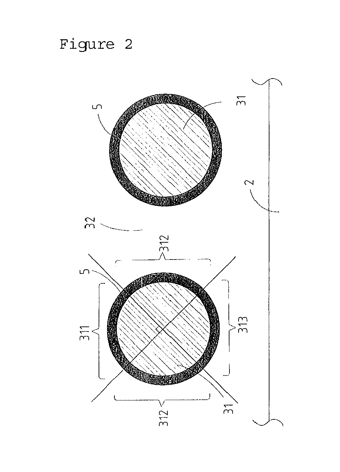

Hereinafter, the guidewire according to the first embodiment of the present invention will be described with reference to Figs. 1 and 2.

Fig. 1 is a vertical cross-sectional view showing a guidewire according to an embodiment of the present invention. Fig. 2 is a partially enlarged view of the part A shown in Fig. 1.

It is to be noted that, in Fig. 1, the left side is referred to as "a proximal side," and the right side is referred to as "a front side" for convenience of explanation.

Also, in Fig. 1, the guidewire is shortened in the longitudinal direction to show the entirety schematically for ease of understanding. Accordingly, the scale ratio of the entire guidewire differs from the actual one.

In Fig. 1, a guidewire 1 has a core shaft 2 tapered toward the front end and a coiled body 3 covering the tip portion of the core shaft 2. The front end of the core shaft 2 and the front end of the coiled body 3 are fixed at a most distal portion 4. Also, the core shaft 2 and the coiled body 3 are fixed at brazed portions 9 (two locations) at positions on the proximal side from the most distal portion 4.

A material constituting the core shaft 2 is not particularly limited. The material constituting the core shaft 2 can be selected from a stainless steel alloy, a super elastic alloy, a cobalt alloy, a piano wire, and tungsten, for example.

A material constituting the coiled body 3 fixed at the tip portion of the core shaft 2 can be selected from radiopaque metals such as platinum, gold, and tungsten and radiolucent metals such as a stainless steel alloy, a super elastic alloy, a cobalt alloy, and a piano wire, for example.

Examples of a material constituting the most distal portion 4 fixing the front end of the core shaft 2 and the front end of the coiled body 3 and the brazed portions 9 include an aluminum alloy brazing material, a silver brazing material, a gold brazing material, zinc, an Sn-Pb alloy, a Pb-Ag alloy, and an Sn-Ag alloy.

As shown in Fig. 1, the surfaces of coils of strand 31 forming the coiled body 3 are coated with a hydrophilic coating agent 5. The surfaces of the coils of strand 31 are coated with the hydrophilic coating agent 5 so that a gap 32 may be formed between the coils of strand 31. The coils of strand 31 forming the coiled body 3 and coated with the hydrophilic coating agent 5 may be wound around the outer circumference of the core shaft 2 with a defined pitch so that adjacent coils of strand 31 are spaced apart by the gap 32.

The coil of strand 31 coated with the hydrophilic coating agent 5 will be described in detail with reference to Fig. 2. The coil of strand 31 has an outward surface portion 311 corresponding to a surface directing outward, an inward surface portion 313 corresponding to a surface directing inward, and side surface portions 312 corresponding to side surfaces between the outward surface portion 311 and the inward surface portion 313. In the present embodiment, the surface portions 311 and 313 and the side surface portions 312 are defined as follows. That is, an intersecting point of two virtual lines made by tilting a line parallel to the central axis of the core shaft 2 45° clockwise and counterclockwise is overlapped on the center of the cross-section of the coil of strand. By doing so, the cross-section of the coil of strand is partitioned into four areas by the two virtual lines. Among the four areas, an area most distant from the core shaft 2 is defined as the outward surface portion 311. Also, among the four areas, an area closest to the core shaft 2 is defined as the inward surface portion 313. Further, among the four areas, two areas other than the outward surface portion 311 and the inward surface portion 313 are defined as the side surface portions 312.

As described above, the coils of strand 31 are coated with the hydrophilic coating agent 5 so that the gap 32 may be formed between the side surface portions 312 of the adjacent coils of strand 31,

Also, the outward surface portion 311, the side surface portions 312, and the inward surface portion 313 are coated with the hydrophilic coating agent 5. The hydrophilic coating agent 5 coating the outward surface portion 311, the side surface portions 312, and the inward surface portion 313 is even in thickness.

In this manner, in the guidewire of the first embodiment, the coils of strand 31 are coated with the hydrophilic coating agent 5 so that the gap 32 may be formed between the side surface portions 312 of the adjacent coils of strand 31. Accordingly, the movement of the coils of strand 31 is not inhibited, and flexibility of the coiled body 3 is secured although the coils of strand 31 are coated with the hydrophilic coating agent 5.

The coils of strand 31 are coated with the hydrophilic coating agent 5 so that the gap 32 may be formed. Thus, when the guidewire 1 is sterilized by gas such as ethylene oxide, the gas flows into the coil from the gaps 32 of the coiled body 3. Consequently, the inside of the coiled body 3 can be sterilized effectively. In addition, the gas can be exhausted easily after the sterilization.

Meanwhile, at a distal portion 35 (refer to Fig. 1) of the coiled body 3, the coils of strand 31 can be coated with the hydrophilic coating agent 5 so that the gap 32 may be formed between the coils of strand 31.

In this case, the gaps 32 are formed at the distal portion 35 of the coiled body 3. Thus, the distal portion 35 of the coiled body 3 can obtain lubricity due to hydrophilicity and can secure flexibility.

Alternatively, the gaps 32 may be formed over the entire length of the coiled body 3. In this case, the entire length of the coiled body 3 becomes flexible. Thus, flexibility of the entire coiled body 3 can be secured more reliably.

Examples of the material for the hydrophilic coating agent 5 coating the surfaces of the coils of strand 31 include nonionic hydrophilic polymers such as polyvinyl alcohol, polyvinyl pyrrolidone, polyethylene glycol, polyacrylamide, polymethylacrylamide, poly (2-hydroxyethyl methacrylate), and poly (N-hydroxyethyl acrylamide), anionic hydrophilic polymers such as polyacrylic acid, polymethacrylic acid, polymaleic acid, carboxymethyl cellulose, hyaluronic acid, and poly (2-acrylamide-2-methylpropanesulfonic acid), and cationic hydrophilic polymers such as polyethyleneimine, polyallylamine, and polyvinylamine. Also, plural kinds of these hydrophilic polymers may be used as the materials for the hydrophilic coating agent 5. It is to be noted that, in a case of using plural kinds of ionic functional groups as the materials for the hydrophilic coating agent 5, it is preferable to prevent an ion complex from being formed. Thus, in the case of using ionic functional groups, it is preferable to use only either anionic or cationic functional groups.

Also, as the material for the hydrophilic coating agent 5, a polymer obtained by conducting radical polymerization of a monomer constituting the aforementioned polymer may be used. The polymerization of a monomer facilitates control of the film thickness. Further, combination of several kinds of monomers enables control of hydrophilicity of the copolymer. A preferred polymerization when conducted is a living radical polymerization in which the molecular weight distribution is easily controlled such as a nitroxyl method, an atom transfer radical polymerization, or a reversible addition-fragmentation chain transfer polymerization. By using the living radical polymerization method, the thickness of the hydrophilic coating agent 5 on the coils of strand 31 can be controlled easily.

Still further, as the material for the hydrophilic coating agent 5, a mixture of a hydrophilic polymer and a hydrophobic polymer may be used. In this case, when the hydrophilic coating agent 5 comes into contact with a fluid or a body fluid such as blood, the liquid is removed from the hydrophobic polymer and is collected to the hydrophilic polymer by the action of the hydrophobic polymer. Accordingly, lubricity of the hydrophilic coating agent 5 can be maintained.

Also, by using a copolymer of a hydrophilic monomer and a hydrophobic monomer as the hydrophilic polymer for the hydrophilic coating agent 5, lubricity of the hydrophilic coating agent 5 can be maintained for the same reason as that in the case of using the mixture of the hydrophilic polymer and the hydrophobic polymer.

Still further, as the material for the hydrophilic coating agent 5, a hydrophilic polymer including an ionic polymer made of either anions or cations may be used. In this case, when the hydrophilic coating agent 5 comes into contact with a fluid or a body fluid such as blood, the hydrophilic polymers are to be dispersed by electrostatic repulsion of the ionic polymers themselves. Accordingly, flexibility of the hydrophilic coating agent 5 is improved, and flexibility of the coiled body 3 coated with the hydrophilic coating agent 5 can be secured more reliably. Also, a narrow space is generated between the hydrophilic polymers that are to be dispersed, and water molecules flow into this space. Accordingly, water retentivity of the hydrophilic coating agent 5 is improved, and lubricity of the hydrophilic coating agent 5 can be maintained.

Also, to maintain hydrophilicity of the aforementioned hydrophilic coating agent 5, cross-linked hydrophilic polymer gel can be used as the material for the hydrophilic coating agent 5.

By gelling of the hydrophilic polymer, a fluid can be stored inside the gel. Accordingly, lubricity of the hydrophilic coating agent 5 can be maintained. Also, the gelling contributes to increase in mechanical strength of the hydrophilic coating agent 5. Accordingly, the hydrophilic coating agent 5 can be prevented from being peeled.

A state of the hydrophilic polymer in the hydrophilic coating agent 5 used in the present invention can be selected from a state in which the straight-chain, branched, or spherical (including dendrimer) hydrophilic polymers are fixed on the coiled body 3 and such a state as a polymer brush in which one end of each hydrophilic polymer is fixed on the metal surface of the coiled body 3, for example.

Among other things, the especially preferable state of the hydrophilic polymer is the polymer brush state. By using the hydrophilic polymer in the polymer brush state, the molecular weight is easily controlled. Accordingly, the gaps 32 of the coiled body 3 can be formed effectively, and thus flexibility of the coiled body 3 can be secured reliably.

Examples of a method for fixing the straight-chain, branched, or spherical (including dendrimer) hydrophilic polymers on the coiled body 3 are known methods such as a method for immersing the coiled body 3 in a solution in which the aforementioned hydrophilic polymers are dissolved, a method for coating the coiled body with the solution with use of a brush, and a method by spray coating.

A solvent used to prepare the solution in which the aforementioned hydrophilic polymers are dissolved only needs to be a highly polar solvent. The solvent can be selected from water, methanol, ethanol, N-propanol, acetonitrile, isopropyl alcohol, dimethyl sulfoxide, dimethyl acetamide, dimethyl formamide, and N-methyl-2-pyrrolidone, for example.

Also, examples of a method for forming the aforementioned polymer brush are known techniques referred to as a grafting-from method and a grafting-to method. The method for forming the polymer brush is not particularly limited. At the time of forming the polymer brush, the aforementioned methods can be selectively used in accordance with their respective functional characteristics.

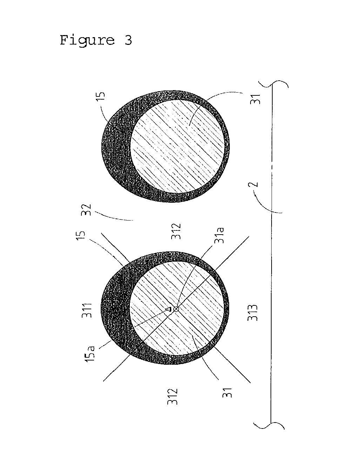

Next, a guidewire according to a second embodiment of the present invention will be described with reference to Fig. 3 mainly on the difference from the first embodiment.

Fig. 3 is a partially enlarged view of the coils of strand 31 coated with a hydrophilic coating agent 15.

As shown in Fig. 3, the coils of strand 31 are coated with the hydrophilic coating agent 15 so that the gap 32 may be formed between the side surface portions 312 of the adjacent coils of strand 31.

The hydrophilic coating agent 15 coats the outer circumference of the coil of strand 31 so that a center 15a of a cross-section formed by the coil of strand 31 and the hydrophilic coating agent 15 may be displaced further in a direction of the outward surface portion 311 than a center 31a of a cross-section of the coil of strand 31. The thickness of the hydrophilic coating agent 15 becomes smaller in the order of the outward surface portion 311, the side surface portions 312, and the inward surface portion 313.

In the guidewire of the second embodiment, the hydrophilic coating agent 15 is protruded in the direction of the outward surface portion 311. In such a shape, the coating thickness of the hydrophilic coating agent 15 is increased at the outward surface portion 311. Accordingly, since hydrophilicity is given to the guidewire for a long period, durability of lubricity of the guidewire 1 can be improved.

Also, since the thickness of the hydrophilic coating agent 15 coating the inward surface portion 313 is small, a space between the core shaft and the coiled body 3 can be secured sufficiently. Accordingly, flexibility of the coiled body 3 can be further improved.

Such a shape of the hydrophilic coating agent 15 can be formed by the following method, for example.

Before coating the coils of strand 31 with the hydrophilic coating agent 15, to heighten affinity of the coils of strand 31 for the hydrophilic coating agent 15, it is preferable to irradiate the outer circumference of the coiled body 3 with ultraviolet to activate the surfaces of the coils of strand 31.

When ultraviolet is irradiated from the outside of the coiled body 3, the outward surface portion 311 of the coil of strand 31 is most activated, and the side surface portions 312 are second most activated. The amount of ultraviolet to be irradiated to each side surface portion 312 is less than that to be irradiated to the outward surface portion 311. Thus, the activation level of the side surface portion 312 is lower than that of the outward surface portion 311. The inward surface portion 313 is not activated since the irradiated light does not reach it. Due to such differences in activation, the activation level of the side surface portion 312 decreases from the side of the outward surface portion 311 toward the side of the inward surface portion 313.

When the coiled body 3 activated in such a manner is coated with the hydrophilic coating agent 15, the larger amount of the hydrophilic coating agent 15 is attached to a more activated portion. Thus, the density of the hydrophilic coating agent 15 becomes lower in the order of the outward surface portion 311, the side surface portions 312, and the inward surface portion 313. The thickness of the hydrophilic coating agent 15 also changes in accordance with the differences in density. Also, the density of the hydrophilic coating agent 15 at the side surface portion 312 gradually decreases from the side of the outward surface portion 311 toward the side of the inward surface portion 313. The film thickness of the hydrophilic coating agent 15 there also decreases in a similar manner.

As a result, lubricity of the hydrophilic coating agent 15 at the outward surface portion 311 is greater than those of the side surface portions 312 and the inward surface portion 313. Also, lubricity of the hydrophilic coating agent 15 at the outward surface portion 311 can be maintained for a long period.

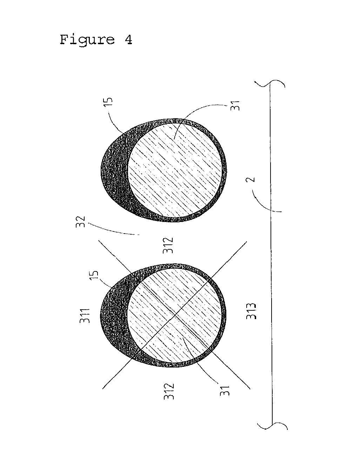

Also, in a modification example of the second embodiment, the side surface portions 312 of the coil of strand 31 may be shielded by a shielding member made of a resin or a metal before irradiation of ultraviolet. By irradiating the coil of strand 31 with ultraviolet thereafter, the hydrophilic coating agent 15 at the side surface portion 312 can be as thin as that at the inward surface portion 313, as shown in Fig. 4. This enables the gaps 32 of the coiled body 3 to be formed further efficiently. Accordingly, flexibility of the coiled body 3 can be secured further reliably.

Meanwhile, only a part of the coils of strand 31 in the direction of the long axis of the guidewire 1 may be coated with the hydrophilic coating agent 5 or 15 shown in Figs. 1 to 4. Alternatively, the entire coils of strand 31 extending in the direction of the long axis of the guidewire 1 may be coated with the hydrophilic coating agent 5 or 15.

While the invention has been shown and described in detail, the foregoing description is in all aspects illustrative and not restrictive. It is therefore understood that numerous modifications and variations can be devised without departing from the spirit and scope of the invention.

### Reference Signs List

- 1: guidewire
- 2: core shaft
- 3: coiled body
- 31: coil of strand
- 31a: center of a cross-section of a coil of strand
- 311: outward surface portion
- 312: side surface portion
- 313: inward surface portion
- 4: most distal portion
- 5, 15: hydrophilic coating agent
- 15a: center of a cross-section of a coil of strand and a hydrophilic coating agent

## Claims

1. A guidewire (1) comprising:
a core shaft (2);
a coiled body (3) including coils of strand wound around an outer circumference of the core shaft (2); and
a hydrophilic coating agent (5; 15) coating at least a part of the coiled body (3), **characterized in that**
at least the part of the coiled body (3) is coated with the hydrophilic coating agent (5; 15) so that adjacent coils of strand (31) coated with the hydrophilic coating agent (5; 15) are spaced apart from each other by a gap (32) which prevents a contact of the coated surfaces of the adjacent coils of strand (31).

2. The guidewire (1) according to claim 1, wherein a center (15a) of a combined cross-section formed by the coil of strand (31) and the hydrophilic coating agent (15) is displaced further in a radial outward direction of the coiled body (3) than a center (31a) of a cross-section of the coil of strand (31) as such.

3. The guidewire (1) according to claim 1 or 2, wherein a thickness of the hydrophilic coating agent (15) coating the coil of strand (31) in an axial direction of the coiled body (3) is smaller than a thickness of the hydrophilic coating agent (15) in a radial outward direction of the coiled body (3).

4. The guidewire (1) according to any one of claims 1 to 3, wherein a thickness of the hydrophilic coating agent (15) coating the coil of the strand (31) in a radial inward direction of the coiled body (3) is smaller than a thickness of the hydrophilic coating agent (15) in an axial direction of the coiled body (3).
